(19) Europäisches Patentamt
European Patent Office
Office européen des brevets

(11) **EP 3 252 455 A1**

(12) **DEMANDE DE BREVET EUROPEEN**

(43) Date de publication:
**06.12.2017 Bulletin 2017/49**

(51) Int Cl.:
***G01N 21/45*** (2006.01)          ***G03H 1/00*** (2006.01)
***G03H 1/08*** (2006.01)

(21) Numéro de dépôt: **16305625.2**

(22) Date de dépôt: **30.05.2016**

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**
Etats d'extension désignés:
**BA ME**
Etats de validation désignés:
**MA MD**

(71) Demandeurs:
• **Biomérieux**
  **69280 Marcy L'etoile (FR)**

• **Bioaster**
  **69007 Lyon (FR)**

(72) Inventeurs:
• **DOUET, Alice**
  **16320 VILLEBOIS-LAVALETTE (FR)**
• **JOSSO, Quentin**
  **69007 LYON (FR)**

(74) Mandataire: **Cabinet Laurent & Charras**
  **Le Contemporain**
  **50 Chemin de la Bruyère**
  **69574 Dardilly Cedex (FR)**

(54) **DISPOSITIF ET PROCEDE D'ACQUISITION D'UNE PARTICULE PRESENTE DANS UN ECHANTILLON**

(57)    L'invention concerne un dispositif d'acquisition (10) de particules (11a-11f) présentes dans un échantillon (12), ledit dispositif d'acquisition comportant une source lumineuse (15) spatialement cohérente, une optique et un capteur d'image (16) placé dans le plan focal de l'optique et configuré pour capturer une image en intensité, et une unité informatique configurée pour :
- construire une série de matrice électromagnétique de propagation obtenues pour une pluralité d'écarts de défocalisation par rapport à un plan de mise au point de l'optique ;
- déterminer une première matrice électromagnétique de focalisée moyenne sur les particules à partir de la série de matrices électromagnétique,
- identifier au moins une des particules dans la première matrice électromagnétique et de mémorisation des coordonnées de ladite particule ; et
- déterminer une seconde matrice électromagnétique à une distance de mise au point sur une particule identifiée à partir des composantes de la série de matrices électromagnétique ayant les coordonnées mémorisées.

Fig. 1

**Description**

<u>DOMAINE TECHNIQUE</u>

La présente invention concerne le domaine de l'acquisition optique de particules biologiques. Les particules biologiques peuvent être des microorganismes tels que des bactéries, des champignons ou des levures par exemple. Il peut également s'agir de cellules, organismes multicellulaires, ou toute autre particule de type particule polluante, poussière.

L'invention trouve une application particulièrement avantageuse pour analyser l'état d'une particule biologique, par exemple pour savoir l'état métabolique d'une bactérie suite à l'application d'un antibiotique. L'invention permet, par exemple, de réaliser un antibiogramme d'une bactérie.

<u>ART ANTERIEUR</u>

Un antibiogramme est une technique de laboratoire visant à tester le phénotype d'une souche bactérienne vis-à-vis d'un ou plusieurs antibiotiques. Un antibiogramme est classiquement réalisé par culture d'un échantillon contenant des bactéries et un antibiotique.

La demande de brevet européen N° 2 603 601 décrit une méthode pour réaliser un antibiogramme en visualisant l'état des bactéries après une durée d'incubation en présence d'un antibiotique. Pour visualiser les bactéries, les bactéries sont marquées par des marqueurs fluorescents permettant de révéler leurs structures. La mesure de la fluorescence des marqueurs permet alors de déterminer si l'antibiotique a agi efficacement sur les bactéries.

Cependant, le processus de marquage est particulièrement long et complexe à réaliser et ces marqueurs chimiques ont un effet cytotoxique sur les bactéries. Il s'ensuit que ce mode de visualisation ne permet pas d'observer les bactéries à plusieurs instants de la culture des bactéries.

Il est donc nécessaire d'utiliser un temps de culture suffisamment long, de l'ordre de 24 à 72 heures, pour garantir la fiabilité de la mesure.

D'autres méthodes de visualisation de particules biologiques utilisent un microscope, permettant une mesure non destructive d'un échantillon.

La microscopie holographique numérique ou DHM (Digital Holographie Microscopy) est une technique d'imagerie permettant de s'affranchir des contraintes de profondeur de champ de la microscopie optique classique. Schématiquement, elle consiste à enregistrer un hologramme formé par l'interférence entre les ondes lumineuses diffractées par l'objet observé et une onde de référence présentant une cohérence spatiale. Cette technique est décrite dans l'article de revue de Myung K.Kim intitulé « Principles and techniques of digital holographie microscopy» publié dans SPIE Reviews Vol. 1, N°1, Janvier 2010.

Récemment, il a été proposé d'utiliser la microscopie holographique numérique pour identifier des microorganismes de manière automatisée. Ainsi, l'article de N. Wu et al. intitulé « Three-dimensional identification of microorganisms using a digital holographic microscope» publié dans Computational and Mathematical Methods in Medicine, Vol. 2013, art. N° ID 162105, décrit une méthode d'identification de différents types de bactéries dans le volume à analyser grâce à une propagation numérique vers le plan de mise au point de la particule. Les images mises au point à différentes profondeurs sont utilisées pour reconstituer une représentation tridimensionnelle des microorganismes. Ceux-ci sont alors classifiés au moyen d'un filtrage 3D non linéaire.

De manière similaire, l'article de Ahmed El Mallahi intitulé « Automated threedimensional detection and classification of living organisms using digital holography microscopy with partial spatial coherent source: application to monitoring of drinking water resources » publié dans Applied Optics, Vol. 52 N° 1, Janvier 2013, décrit une méthode comprenant une première étape de détection de la position des bactéries dans le volume à analyser, une étape de focalisation à différentes profondeurs du volume au moyen d'une propagation numérique, puis une classification des bactéries à partir de leurs caractéristiques morphologiques.

Les méthodes d'identification précitées sont toutefois complexes dans la mesure où elles nécessitent une focalisation dans des plans successifs de mise au point.

A contrario, la focalisation dans un seul plan de mise au point, autrement dit à une seule profondeur d'analyse, ne suffit généralement pas à identifier un type de microorganisme avec un faible taux de fausse détection.

Le problème technique objectif de la présente invention est, par conséquent, d'observer une particule biologique en limitant le temps d'acquisition, c'est-à-dire sans marquage et sans une mise au point précise du système optique.

<u>EXPOSE DE L'INVENTION</u>

Pour ressoude le problème technique, l'invention propose un dispositif d'acquisition d'une particule intégrant une simple acquisition sans focalisation associée à une reconstruction numérique de la focalisation comportant : une première focalisation moyenne destinée à détecter au moins une particule et une seconde focalisation spécifique d'une région d'intérêt contenant la particule.

A cet effet, selon un premier aspect, l'invention concerne un dispositif d'acquisition d'une pluralité de particules présentes dans un échantillon, ledit dispositif d'acquisition comportant :

- une source lumineuse, spatialement cohérente ou pseudo-cohérente, orientée sur une première face dudit échantillon ;
- un système optique ayant un axe optique et réalisant la conjugaison entre un plan de mise au point et un plan focal, orienté sur une seconde face dudit échantillon opposé à ladite première face, et placé par rapport à l'échantillon de sorte que les particules ne sont pas dans le plan de mis au point ;
- un capteur d'image, placé dans le plan focal du système optique et configuré pour acquérir une image en intensité formée par l'interférence entre ladite source lumineuse et ledit échantillon ; et
- une unité de traitement informatique comportant :

  o un module de construction numérique d'une série de matrices électromagnétiques modélisant, à partir de l'image acquise, l'onde électromagnétique dans des plans parallèles au plan de mise au point et compris dans l'échantillon pour une pluralité d'écarts par rapport audit plan ;
  o un module de détermination d'une première matrice électromagnétique à une distance de mise au point moyenne sur les particules à partir de la série de matrices électromagnétiques ;
  o un module d'identification d'au moins une des particules dans la première matrice électromagnétique et de mémorisation des coordonnées de ladite particule ; et
  o un module de détermination d'une seconde matrice électromagnétique à une distance de mise au point sur une particule identifiée à partir des composantes de la série de matrices électromagnétique ayant les coordonnées mémorisées.

L'invention permet ainsi d'observer des phénomènes similaires à ceux décrits dans l'état de l'art sans marquage chimique. La mise au point est effectuée de manière numérique à partir d'une image défocalisée associée à une reconstruction numérique de la focalisation comportant : une première focalisation moyenne destinée à détecter au moins une particule et une seconde focalisation spécifique d'une région d'intérêt contenant la particule.

Il s'ensuit que les instruments de mesure sont simplifiés car il n'est pas nécessaire d'utiliser des appareils de mise au point extrêmement précis permettant de mettre au point une image de quelques nanomètres. Le temps d'acquisition est également réduit car la mise au point ou le marquage n'est plus nécessaire.

En outre, les opérations de marquage et de mise au point sont classiquement réalisées manuellement. L'invention permet également de limiter ces interactions manuelles durant l'acquisition et donc d'automatiser le processus d'acquisition d'une particule. Il s'ensuit que le dispositif d'acquisition d'une particule présente dans un échantillon peut être positionné au plus près d'un patient de sorte à améliorer la rapidité de traitement d'un patient.

Le processus classique pour déterminer les antibiotiques efficaces sur une souche bactérienne consiste à réaliser un prélèvent contenant ladite souche (e.g. sur un patient, un animal, un lot alimentaire,...) puis à transmettre le prélèvement à un centre d'analyse. Lorsque le centre d'analyse réceptionne le prélèvement, il procède tout d'abord à la culture de la souche bactérienne pour obtenir au moins une colonie de celle-ci, culture comprise entre 24 heure et 72 heures. Il prépare ensuite à partir de cette colonie plusieurs échantillons comprenant des antibiotiques différents et/ou des concentrations d'antibiotiques différentes, puis met à nouveau les échantillons à incuber. Après une nouvelle durée de culture comprise également entre 24 et 72 heures, chaque échantillon est analysé manuellement pour déterminer si l'antibiotique a agi efficacement. Les résultats sont alors retransmis au praticien pour appliquer l'antibiotique et/ou la concentration d'antibiotique le plus efficace. L'invention permet de refondre entièrement ce processus en déplaçant le dispositif d'analyse proche du praticien car la manipulation subtile d'un opérateur n'est plus nécessaire pour effectuer la mise au point ou le marquage.

Typiquement, l'invention a permis de détecter les modifications structurelles d'une bactérie en présence d'un antibiotique après une incubation de seulement une dizaine de minute, et sa sensibilité au bout de deux heures (détection de la présence ou de l'absence d'une division ou d'un motif codant la division) contrairement au processus précédemment décrit qui peut prendre plusieurs jours. En effet, les mesures étant non destructives, il est possible de réaliser des analyses très tôt dans le processus de culture sans risquer de détruire l'échantillon et donc de prolonger le temps d'analyse.

Selon un mode de réalisation, ledit capteur d'image est configuré pour acquérir un flux d'images, et l'unité de traitement est configurée pour suivre une particule dans le flux de premières matrices électromagnétiques.

Ce mode de réalisation permet de suivre une particule sur plusieurs images successives de sorte à former un film représentant l'évolution d'une particule au cours du temps. La méthode classique du marquage chimique ne permet pas de représenter l'évolution d'une particule au cours du temps car les particules sont altérées après la première analyse.

La méthode classique de visualisation de particules biologiques utilisant la microscopie classique (non holographique) permet de représenter une particule au cours du temps mais elle nécessite une focalisation manuelle pour chaque image. En effet, une particule n'est pas fixe dans un échantillon et elle peut se déplacer dans le plan du support ou dans la profondeur de l'échantillon. Même si une particule se déplace uniquement dans le plan de son support, la focalisation de la particule peut être défaillante entre deux images en raison d'un défaut de planéité du support.

Ce mode de réalisation permet de représenter une particule au cours du temps en recherchant une focalisation pouvant varier selon le plan du support ou dans la profondeur de l'échantillon. Avantageusement les particules sont des microorganismes, et la durée entre deux images acquises issues dudit capteur d'image est inférieure ou égale à 1 minute.

Typiquement, ce mode de réalisation de l'invention a permis d'observer la division cellulaire d'une bactérie.

Il s'ensuit que ce mode de réalisation permet d'augmenter la fiabilité et la rapidité d'analyse de l'état d'une particule. En effet, au lieu d'analyser une particule uniquement en fonction de ses caractéristiques physiologique, il est possible d'étudier une particule en fonction de son comportement. Par exemple, il est possible de constater qu'une bactérie ne se divise plus lorsqu'un antibiotique efficace est présent dans l'échantillon.

Selon un mode de réalisation, ladite série de matrices électromagnétiques est obtenue par un modèle de propagation numérique d'une lumière à travers ledit échantillon, les matrices électromagnétiques variant en modulant une distance d'un axe optique dudit modèle de propagation. Ce mode de réalisation permet de reconstruire efficacement l'image de la particule.

Avantageusement, l'unité de traitement comprend un module de transformation des matrices électromagnétiques issues du modèle de propagation par une application surjective de l'espace complexe dans l'espace réel. Les matrices obtenues permettent une visualisation directement compréhensible par un opérateur ainsi que la mise en oeuvre de traitement d'image classique sur la base par exemple de la morphologie des particules.

Selon un mode de réalisation, la première matrice électromagnétique est obtenue en représentant, à chaque coordonnée, les composantes à ladite coordonnée des matrices électromagnétiques en fonction de ladite distance dudit axe optique, et en recherchant une moyenne des maxima de toutes les représentations. Ce mode de réalisation permet de détecter efficacement la matrice focalisée moyenne.

Selon un mode de réalisation, la seconde image électromagnétique est obtenue en représentant, à chaque coordonnée de la particule identifiée, les composantes à ladite coordonnée des matrices électromagnétiques en fonction de ladite distance dudit axe optique, et en recherchant une moyenne des maxima de toutes les représentations. Ce mode de réalisation permet de détecter efficacement la matrice focalisée sur la particule.

Selon un mode de réalisation, les matrices de ladite série de matrices électromagnétiques utilisées pour déterminer ladite première matrice électromagnétique et/ou pour déterminer ladite seconde matrice électromagnétique sont sous-échantillonnées selon ladite distance. Ce mode de réalisation permet de limiter le temps de calcul nécessaire pour les focalisations.

Selon un mode de réalisation, ledit dispositif comporte plusieurs unités d'acquisition, chaque unité d'acquisition comportant un capteur d'image et des moyens de focalisation spécifiques, ledit dispositif étant configuré pour représenter une image d'une particule de chaque échantillon. Ce mode de réalisation permet de faire des comparaisons rapides entre plusieurs échantillons, par exemple pour réaliser un antibiogramme.

Ce premier aspect de l'invention peut également être formulé sous la forme d'un procédé d'acquisition d'une pluralité de particules présentes dans un échantillon, ledit procédé d'acquisition comportant les étapes suivantes :

- émission d'une source lumineuse, spatialement cohérente ou pseudo-cohérente, orientée sur une première face dudit échantillon ;
- acquisition à l'aide d'un capteur d'image d'une image en intensité, ledit capteur étant placé dans le plan focal d'un système optique ayant un axe optique et réalisant la conjugaison entre un plan de mise au point et le plan focal, orienté sur une seconde face dudit échantillon opposé à ladite première face, et placé par rapport à l'échantillon de sorte que la particule n'est pas dans le plan de mis au point, l'image étant formée par interférence entre ladite source lumineuse et ledit échantillon ;

- construction numérique d'une série de matrices électromagnétiques modélisant, à partir de l'image acquise, l'onde électromagnétique dans des plans parallèles au plan de mise au point et compris dans l'échantillon pour une pluralité d'écarts par rapport audit plan ;
- identification d'au moins une des particules dans la première matrice électromagnétique et mémorisation des coordonnées de ladite particule ; et
- détermination d'une seconde matrice électromagnétique à une distance de mise au point sur une particule identifiée à partir des composantes de la série de matrices électromagnétique ayant les coordonnées mémorisées.

Selon un second aspect, l'invention a également pour objet un procédé d'analyse d'au moins une particule présente dans un échantillon, ledit procédé d'analyse comportant les étapes suivantes :

- émission d'une source lumineuse, spatialement cohérente ou pseudo-cohérente, orientée sur une première face dudit échantillon ;
- acquisition à l'aide d'un capteur d'image d'une image en intensité, ledit capteur étant placé dans le plan focal d'un système optique ayant un axe optique et réalisant la conjugaison entre un plan de mise au point et le plan focal, orienté sur une seconde face dudit échantillon opposé à ladite première face, et placé par rapport à l'échantillon de sorte que la particule n'est pas dans le plan de mis au point, l'image étant formée par interférence entre ladite source lumineuse et ledit échantillon ;
- construction numérique d'une série de matrices électromagnétiques modélisant, à partir de l'image acquise, l'onde électromagnétique dans des plans parallèles au plan de mise au point et compris dans l'échantillon pour une pluralité d'écarts par rapport audit plan ;
- obtention d'une matrice électromagnétique à une distance de mise au point sur la particule à partir de la série de matrices électromagnétique ; et
- détermination d'un état de ladite particule en fonction de la matrice électromagnétique à la distance de mise au point sur la particule.

Selon ce second aspect, l'invention permet également d'observer des phénomènes similaires à ceux décrits dans l'état de l'art sans marquage chimique.

La mise au point est effectuée de manière numérique à partir d'une image défocalisée associée à une reconstruction numérique de la focalisation. Pour finir, l'état de la particule est déterminé à partir de l'image focalisée. Il s'ensuit que les instruments de mesure sont simplifiés car il n'est pas nécessaire d'utiliser des appareils de mise au point extrêmement précis permettant de mettre au point une image de quelques nanomètres. Le temps d'acquisition est également réduit car la mise au point ou le marquage n'est plus nécessaire.

En outre, les opérations de marquage et de mise au point sont classiquement réalisées manuellement. Selon ce second aspect, l'invention permet également de limiter ces interactions manuelles durant l'acquisition et donc d'automatiser le processus d'analyse d'une particule. Il s'ensuit que le procédé d'analyse d'une particule présente dans un échantillon peut être réalisé au plus près d'un patient de sorte à améliorer la rapidité de traitement d'un patient.

Selon un mode de réalisation, l'étape d'obtention de la matrice électromagnétique à la distance de mise au point sur la particule comporte les étapes suivantes :

- détermination d'une première matrice électromagnétique à une distance de mise au point moyenne sur les particules à partir de la série de matrices électromagnétiques ;
- l'identification de la particule dans la première matrice électromagnétique et la mémorisation des coordonnées de ladite particule ; et
- la détermination de la matrice électromagnétique à la distance de mise au point sur la particule à partir des composantes de la série de matrices électromagnétique ayant les coordonnées mémorisées.

Ce mode de réalisation permet de déterminer efficacement une matrice focalisée de ladite particule au moyen d'une première focalisation moyenne destinée à détecter au moins une particule et d'une seconde focalisation spécifique sur la particule.

Selon un mode de réalisation, ladite étape de détermination d'un état de ladite particule comporte les étapes suivantes :

- rotation de la matrice électromagnétique à la distance de mise au point sur la particule de manière à aligner la particule sur un axe prédéfini, et
- calcul d'une distribution de la matrice alignée selon ledit axe prédéfini.

Ce mode de réalisation permet de s'affranchir de l'orientation et de la position de la particule. En outre, la distribution permet de catégoriser la forme de la particule.

Selon un mode de réalisation, les étapes d'acquisition d'une image holographique jusqu'à l'étape d'obtention d'une image focalisée d'une particule sont réalisées au cours du temps pour plusieurs images, l'étape de détermination d'un état de ladite particule étant réalisée en fonction d'une évolution temporelle de ladite particule.

Ce mode de réalisation permet de représenter une particule au cours du temps en recherchant une focalisation pouvant varier selon le plan du support ou dans la profondeur de l'échantillon. Avantageusement, la durée entre deux images issues dudit capteur d'image est inférieure ou égale à 1 minutes dans le cadre de microorganisme, en particulier de bactéries.

Selon un mode de réalisation, ladite étape de détermination d'un état de ladite particule comporte les étapes suivantes :

- rotation pour chaque image acquise, de la matrice électromagnétique à la distance de mise au point sur la particule de manière à aligner la particule sur un axe prédéfini ; et
- calcul, pour chaque image acquise, d'une distribution de la matrice alignée selon ledit axe prédéfini ; et
- représentation matricielle des distributions associées à chaque image en fonction du temps.

Ce mode de réalisation permet d'améliorer la catégorisation de la particule.

Selon un mode de réalisation, ladite étape de détermination d'un état de ladite particule est réalisée en fonction d'un état de division de ladite particule.

Ce mode de réalisation permet, par exemple, de détecter si une bactérie est dans un cycle de division ou si un inhibiteur bloque ce cycle de division.

Selon un mode de réalisation, ladite étape de détermination d'un état de ladite particule est réalisée en fonction d'une caractéristique morphologique de ladite particule, par exemple la longueur.

Selon un mode de réalisation, ladite étape de détermination d'un état de ladite particule est réalisée en fonction d'une caractéristique physiologique de ladite particule, par exemple le nombre de noyau.

Selon un mode de réalisation, ladite particule correspond à une bactérie et ledit échantillon intègre un antibiotique.

Ce second aspect de l'invention peut également être formulé sous la forme d'un dispositif d'acquisition d'au moins une particule présente dans un échantillon, ledit dispositif d'acquisition comportant :

- une source lumineuse, spatialement cohérente ou pseudo-cohérente, orientée sur une première face dudit échantillon ;
- un système optique ayant un axe optique et réalisant la conjugaison entre un plan de mise au point et un plan focal, orienté sur une seconde face dudit échantillon opposé à ladite première face, et placé par rapport à l'échantillon de sorte que les particules ne sont pas dans le plan de mis au point ;
- un capteur d'image, placé dans le plan focal du système optique et configuré pour acquérir une image en intensité formée par l'interférence entre ladite source lumineuse et ledit échantillon ; et
- une unité de traitement informatique comportant :

  o un module de construction numérique d'une série de matrices électromagnétiques modélisant, à partir de l'image acquise, l'onde électromagnétique dans des plans parallèles au plan de mise au point et compris dans l'échantillon pour une pluralité d'écarts par rapport audit plan ;
  o un module d'obtention d'une matrice électromagnétique à une distance de mise au point sur la particule à partir de la série de matrices électromagnétique ;
  o un module d'identification d'au moins une des particules dans la première matrice électromagnétique et de mémorisation des coordonnées de ladite particule ; et
  o un module de détermination d'un état de ladite particule en fonction de la matrice électromagnétique à la distance de mise au point sur la particule.

## DESCRIPTION SOMMAIRE DES FIGURES

La manière de réaliser l'invention ainsi que les avantages qui en découlent, ressortiront bien du mode de réalisation qui suit, donné à titre indicatif mais non limitatif, à l'appui des figures annexées dans lesquelles les figures 1 à 6 représentent :

- figure 1 : une représentation schématique en coupe d'un dispositif d'acquisition d'une particule présente dans un échantillon selon un mode de réalisation de l'invention ;
- figure 2 : une représentation schématique d'un organigramme de fonctionnement d'une unité de traitement de la figure 1 selon un premier mode de réalisation ;
- figure 3 : une représentation schématique d'un organigramme de fonctionnement du module de détermination d'une image focalisée moyenne de la figure 2 ;
- figure 4 : une représentation schématique d'un organigramme de fonctionnement du module de détermination d'une région d'intérêt de la figure 2 ;
- figure 5 : une représentation schématique d'un organigramme de fonctionnement d'une unité de traitement de la figure 1 selon un second mode de réalisation ;
- figure 6 : une représentation schématique d'un organigramme de fonctionnement du module de prédiction d'un état d'une particule de la figure 5 ;
- figure 7 : une représentation schématique d'une image d'une matrice de distributions seuillée ;
- figure 8 : une représentation schématique d'un ensemble de vignettes d'image comprenant une bactérie observée au cours du temps grâce à un procédé selon un mode de réalisation de l'invention ; et

- figure 9 : un ensemble de distribution correspondant aux vignettes de la figure 8.

DESCRIPTION DETAILLEE DE L'INVENTION

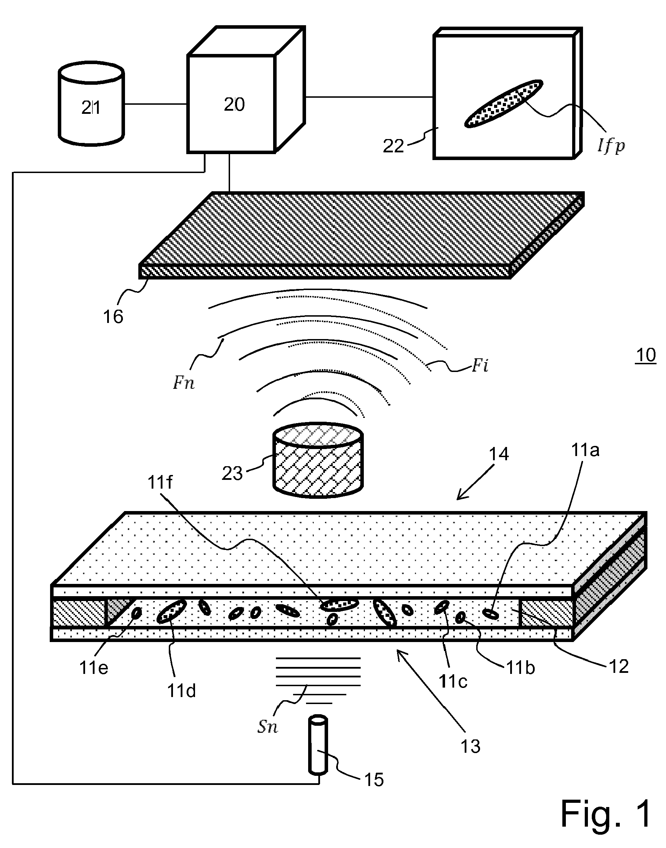

La figure 1 illustre un dispositif d'observation **10** d'une particule **11a-11f** présente dans un échantillon **12**. L'échantillon **12** est disposé entre une source lumineuse **15,** spatialement et temporellement cohérente (e.g. un laser) ou pseudo-cohérente (e.g. une diode électroluminescente, une diode laser), et un capteur numérique **16** sensible dans la gamme spectrale de la source lumineuse. De préférence, la source lumineuse **15** comporte une faible largeur spectrale, par exemple inférieure à 200nm, inférieure à 100nm ou encore inférieure à 25 nm. Dans ce qui suit, il est fait référence à la longueur d'onde d'émission centrale de la source lumineuse, par exemple dans le domaine visible. La source lumineuse **15** émet un signal cohérent *Sn* orienté sur une première face **13** de l'échantillon, par exemple acheminé par un guide d'onde tel qu'une fibre optique.

L'échantillon **12** consiste en un liquide tel que de l'eau, une solution tampon, un milieu de culture ou un milieu réactif (comprenant ou non un antibiotique), dans lequel se trouvent les particules **11a-11f** à observer.

En variante, l'échantillon **12** peut se présenter sous la forme d'un milieu solide, de préférence translucide, tel qu'une gélose agar-agar, dans lequel se trouvent les particules **11a-11f.** L'échantillon **12** peut également être un milieu gazeux. Les particules **11a-11f** peuvent se situer à l'intérieur du milieu ou bien à la surface de l'échantillon **12.**

Les particules **11a-11f** peuvent être des microorganismes tels que des bactéries, des champignons ou des levures. Il peut également s'agir de cellules, organismes multicellulaires, ou toute autre particule de type particule polluante, poussière. La taille des particules **11a-11f** observées varie entre 500nm et plusieurs centaines de $\mu$m, voire quelques millimètres.

L'échantillon **12** est contenu dans une chambre d'analyse, délimitée verticalement par une lame inférieure et une lame supérieure, par exemple des lames de microscope conventionnelles. La chambre d'analyse est délimitée latéralement par un adhésif ou par tout autre matériau étanche. Les lames inférieure et supérieure sont transparentes à la longueur d'onde de la source lumineuse **15,** l'échantillon et la chambre laissant passer par exemple plus de 50% la longueur d'onde de la source lumineuse sous incidence normale sur la lame inférieure.

De préférence, les particules **11a-11f** sont disposées dans l'échantillon **12** au niveau de la lame supérieure. La face inférieure de la lame supérieure comprend à cet effet des ligands permettant d'accrocher les particules, par exemple des polycations (e.g. poly-L-lysine) dans le cadre de microorganismes Ceci permet de contenir les particules dans une épaisseur égale à, ou proche de, la profondeur de champ du système optique, à savoir dans une épaisseur inférieure à 1mm (e.g. lentille à tube), et de préférence inférieure à 100$\mu$m (e.g. objectif de microscope). Les particules **11a-11f** peuvent néanmoins se déplacer dans l'échantillon **12.**

De préférence, le dispositif comprend un système optique **23** constitué, par exemple, d'un objectif de microscope et d'une lentille de tube, disposé dans l'air et à distance fixe de l'échantillon. Le système optique **23** est optionnellement équipé d'un filtre pouvant être situé devant l'objectif ou entre l'objectif et la lentille de tube. Le système optique **23** est caractérisé par son axe optique, son plan d'objet, également dénommé plan de mise au point, à une distance de l'objectif, et son plan d'image, conjugué du plan d'objet par le système optique. En d'autres termes, à un objet situé dans le plan d'objet, correspond une image nette de cet objet dans le plan image, également appelé plan focal. Les propriétés optiques du système **23** sont fixes (e.g. optique à focale fixe). Les plans objet et image sont orthogonaux à l'axe optique.

Le capteur d'image **16** est situé, en regard d'une seconde face **14** de l'échantillon, dans le plan focal ou à proximité de ce dernier. Le capteur, par exemple un capteur CCD ou CMOS, comprend un réseau bidimensionnel périodique de sites élémentaires sensibles, et une électronique de proximité qui règle le temps d'exposition et la remise à zéro des sites, d'une manière connue en soi. Le signal de sortie d'un site élémentaire est fonction de la quantité de rayonnement de la gamme spectrale incident sur ledit site pendant la durée d'exposition. Ce signal est ensuite converti, par exemple par l'électronique de proximité, en point image, ou « pixel », d'une image numérique. Le capteur produit ainsi une image numérique sous forme d'une matrice à *C* colonnes et *L* lignes. Chaque pixel de cette matrice, de coordonnées (c, *l*) dans la matrice, correspond d'une manière connue en soi à une position de coordonnées cartésiennes ($x(c,\ l),\ y(c,\ l)$) dans le plan focal du système optique **23,** par exemple la position du centre du site sensible élémentaire de forme rectangulaire.

Le pas et le facteur de remplissage du réseau périodique sont choisis pour respecter le critère Shannon-Nyquist vis-à-vis de la taille des particules observées, de manière à définir au moins deux pixels par particule. Ainsi, le capteur d'image **16** acquiert une image en transmission de l'échantillon dans la gamme spectrale de la source lumineuse.

L'image *Ih* acquise par le capteur d'image **16** comprend des informations holographiques dans la mesure où elle résulte de l'interférence entre une onde *Fi* diffractée par les particules **11a-11f** et une onde de référence *Fn* ayant traversée l'échantillon sans avoir interagi avec lui. On comprend évidemment, comme décrit plus haut, que dans le cadre d'un capteur CMOS ou CCD, l'image numérique *Ih* acquise et mémorisée dans l'unité **20** est une image en intensité, l'information de phase étant donc ici codée en intensité, avec *Ih* selon la relation :

$$Ih = \begin{pmatrix} ih(1,1) & \dots & ih(c,1) & \dots & ih(C,1) \\ \vdots & \ddots & \vdots & \dots & \vdots \\ ih(1,l) & \dots & ih(c,l) & \dots & ih(C,l) \\ \vdots & \ddots & \vdots & \ddots & \vdots \\ ih(1,L) & \dots & ih(c,L) & \dots & ih(C,L) \end{pmatrix}$$

Alternativement, il est possible de diviser le signal cohérent *Sn* issu de la source lumineuse **15** en deux composantes, par exemple au moyen d'une lame semi-transparente. La première composante sert alors d'onde de référence et la seconde composante est diffractée par l'échantillon **12,** l'image dans le plan image du système optique **23** résultant de l'interférence entre l'onde diffractée et l'onde de référence.

L'image en intensité *Ih* acquise par le capteur d'image **16** n'est pas focalisée sur la particule à observer et l'obtention d'une information focalisée sur la particule est obtenue numériquement par une unité de traitement **20** connectée au capteur d'image **16** pour recevoir les images acquises par ce dernier.

Par « défocalisée », on entend ici qu'il n'y a pas d'intersection entre le plan de mise au point et la particule objet de l'observation.

L'unité de traitement **20** peut correspondre à un ordinateur, un microcontrôleur, une tablette tactile ou un téléphone intelligent, ou de manière générale tout système informatique à base de processeur capable de recevoir des données, traiter ces données en mettant en oeuvre des instructions informatiques stockées dans une mémoire informatique, et de délivrer et/ou stocker dans une mémoire informatique le résultat du traitement. L'unité de traitement **20** peut être connectée de manière filaire ou non filaire avec le capteur d'image **16** ou au moyen d'une communication sans fil. L'unité peut être associée à un écran pour l'affichage des résultats intermédiaires ou finaux du procédé de l'invention.

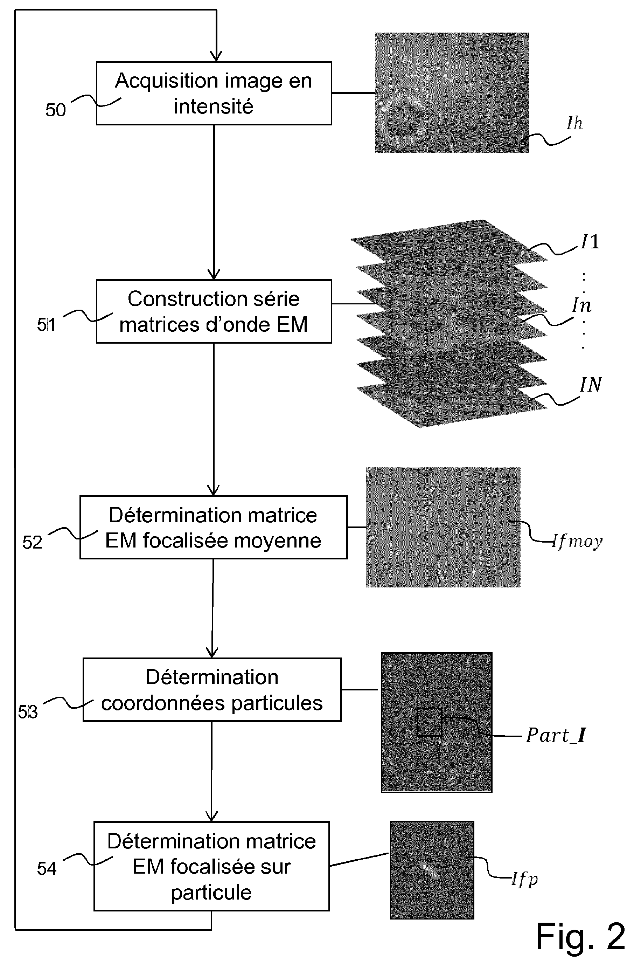

La Figure 2 illustre les modules de traitement informatique **51-53**, intégrés dans l'unité de traitement **20** sous forme d'instructions informatiques mises en oeuvre par l'ordinateur, suite à l'acquisition **50** de l'image en intensité *Ih* par le capteur d'image **16.**

Un premier module **51** construit une série de matrices complexes *I1*, ..., *In*, ...,*IN*, nommées « matrices électromagnétiques » **EM,** modélisant à partir de l'image *Ih* le front d'onde lumineux propagé le long de l'axe optique pour une pluralité d'écarts par rapport au plan de mise au point du système optique **23,** et en particulier des écarts positionnés dans l'échantillon.

Une méthode de calcul de fronts d'onde par propagation numérique est expliqué dans l'article de Sang-Hyuk Lee et al. Intitulé « Holographic microscopy of holographically trapped three-dimensional structures » publié dans Optics Express, Vol. 15 ; N°4, 19 Février 2017, pp. 1505-1512.

Plus précisément, si l'on note $h_z(r)$ la fonction de propagation de Rayleigh-Sommerfeld, soit :

$$h_z(r) = -\frac{1}{2\pi} \frac{\partial}{\partial z} \frac{e^{ikR}}{R}$$

où :

- z est la hauteur dite de « défocalisation », autrement dit l'écart par rapport au plan de mise au point,
- $r = (|r|, \theta)$ est la position en coordonnées polaires dans le plan d'image, de coordonnée radiale $|r|$ et de coordonnées angulaire ,
- $R^2 = |r|^2 + z^2$, et
- $k = 2\pi n/\lambda$ est un nombre d'onde relatif au milieu de propagation d'indice de réfraction n à la longueur d'onde $\lambda$ de la source lumineuse.

A partir de cette relation, l'onde électromagnétique $a(r, z)$, d'amplitude $|a(r, z)|$ et de phase $\varphi(r, z)$, dans le plan d'ordonnée z peut être exprimée sous la forme :

$$a(r,z) = |a(r,z)|\exp(i\varphi(r,z))$$

$$a(r,z) = \frac{1}{4\pi^2} \int_{-\infty}^{+\infty} B(q) H_{-z}(q) \exp(iqr) \, d^2q$$

où

- $b(r)$ est intensité mesurée, i.e. l'image **Ih** (l'intensité de l'onde de référence est ici supposée constante),
- $B(q)$ est la transformée de Fourier de $b(r)$,
- $H_{-z}(q)$ est la transformée de Fourier de $h_{-z}(r)$, et
- $q$ est la variable duale de $r$ dans la transformée de Fourier.

Les équations ci-dessus définissent une formulation analytique de l'amplitude $a(r, z)$. Bien que ce modèle soit développé pour la propagation dans un milieu homogène (et donc sans modification du nombre d'onde, sans présence d'interface source de réflexion et/ou de déviation de l'onde, etc.), et par conséquent sans rapport avec l'échantillon et la chambre (qui comprennent de nombreuses interfaces et changement d'indices par exemple), les inventeurs ont noté qu'il permet de reconstruire des informations électromagnétiques riches en rapport avec les particules observées, comme cela sera décrit-après. Ainsi, avantageusement, l'unité de traitement **20** mémorise un seul nombre d'onde, commun pour tous les milieux en jeux, par exemple l'indice de l'air. En variante, l'unité **20** mémorise les indices de réfraction des différents milieux en jeu le long de l'axe optique et construit les matrices **I1 - IN** de proche en proche pour tenir compte les phénomènes aux interfaces.

Pour des bactéries, le pas d'échantillonnage en z est inférieur de préférence au dixième de l'épaisseur de la bactérie, par exemple inférieure à 0,1 $\mu$m, et de préférence inférieure à 0,03 $\mu$m.

On comprend ainsi que l'on peut construire une pile de matrices électromagnétiques **I1 - IN** pour des ordonnées $z_1, z_2, \ldots, z_n, z_N$ le long de l'axe optique, l'origine des ordonnées ($z = 0$) étant prise à la position axiale de mise au point, chaque matrice **In** étant définie par une amplitude complexe $a(r, z_n)$ selon les relations :

$$\begin{pmatrix} a(1,1)_{z_n} & \ldots & a(c,1)_{z_n} & \ldots & a(C,1)_{z_n} \\ \vdots & \ddots & \vdots & \ldots & \vdots \\ a(1,l)_{z_n} & \ldots & a(c,l)_{z_n} & \ldots & a(C,l)_{z_n} \\ \vdots & \ddots & \vdots & \ddots & \vdots \\ a(L,1)_{z_n} & \ldots & a(c,L)_{z_n} & \ldots & a(C,L)_{z_n} \end{pmatrix}$$

$$\forall (c,l) \in [1,C] \times [1,L] : a(c,l)_{z_n} = a\left(r\big((c,l), y(c,l)\big), z_n\right)$$

L'unité de traitement **20** calcule ensuite sur chaque matrice **In** une application surjective positive $AS$ de l'espace complexe $\mathbb{C}^{C \times L}$ vers l'espace réel $\mathbb{R}^{C \times L}$ :

$$AS(In) = \begin{pmatrix} AS(a(1,1)_{z_n}) & \ldots & AS(a(c,1)_{z_n}) & \ldots & AS(a(C,1)_{z_n}) \\ \vdots & \ddots & \vdots & \ldots & \vdots \\ AS(a(1,l)_{z_n}) & \ldots & AS(a(c,l)_{z_n}) & \ldots & AS(a(C,l)_{z_n}) \\ \vdots & \ddots & \vdots & \ddots & \vdots \\ AS(a(L,1)_{z_n}) & \ldots & AS(a(c,L)_{z_n}) & \ldots & AS(a(C,L)_{z_n}) \end{pmatrix}$$

Par exemple, l'unité **20** calcule la norme hermitienne (ou son carrée) des composantes $a(c, l)_{zn}$, la valeur absolue ou le carré de la partie imaginaire (notée $Im(a(c, l)_{zn})$ ou de la partie réelle (notée $Re(a(c, l)_{zn})$) des composantes $a(c, l)_{zn}$, ou $Re^2(a(c, l)_{zn}) + Im^2(a(c, l)_{zn})$ ou $(Re^2(a(c, l)_{zn}) + Im^2(a(c, l)_{zn}))^{1/2}$.

Sans être lié par la théorie, les matrices **AS(I1) - AS(IN)** ne représentent pas nécessairement une intensité lumineuse, mais les inventeurs ont noté leur ressemblance avec des images en intensité obtenues sous éclairage non cohérent. Notamment, les particules sont représentées, comme dans une photographie, sous leur forme de particules.

Il est ainsi possible d'appliquer tout type de traitement d'image classique (segmentation, seuillage, détection de particule sur la base de leur morphologie, etc.), et même pour un opérateur d'identifier à l'oeil les particules (à la différence d'une image codant des interférences qui sont codées en intensité sous la forme de franges). Dans ce qui suit, pour alléger les notations, les matrices *AS(I1) - AS(IN)* sont notées *I1 - IN,* et la notation $a(c, l)_{zn}$ correspondant à $AS(a(c, l)_{zn})$.

Le procédé selon l'invention consiste ensuite à identifier des particules dans l'échantillon en fonction des matrices *I1 - IN,* et pour chaque particule identifiée, à déterminer une distance *z* de focalisation optimale, i.e. de mise au point, pour cette particule, puis à déterminer dans la matrice de la série *I1 - IN* correspondant à cette distance, un ensemble de pixels appartenant à cette particule.

Le second module **52** vise à déterminer une distance de focalisation moyenne *zfmoy* à partir de la série de matrices *I1 - IN* et à sélectionner dans cette série la matrice, notée *Ifmoy,* dont la distance *z* est égale ou la plus proche de la distance *zfmoy.* En variante, l'unité **20** recalcule la matrice *Ifmoy* pour la distance *zfmoy.* Cette distance de focalisation moyenne *zfmoy* est celle qui correspond au mieux aux conditions idéales de mise au point sur l'ensemble des particules **11a-11f** au sens d'un critère de focalisation prédéterminé. Cette distance peut être déterminée par toutes les techniques connues de traitement du signal ou du domaine de la photographie, par exemple de l'autofocus. La matrice électromagnétique résultante *Ifmoy* est suffisamment « focalisée » pour des particules à différentes profondeurs puissent être détectées dans la matrice *Ifmoy.* Les particules détectées sont notamment celles comprises dans une profondeur d'échantillon égale à la profondeur de champ. Comme décrit précédemment, dans un mode de réalisation préféré, les particules sont disposées dans un volume d'épaisseur proche ou égale à cette profondeur de champ, de sorte que la totalité, ou la quasi-totalité, des particules de l'échantillon peuvent être détectées dans la matrice *Ifmoy.*

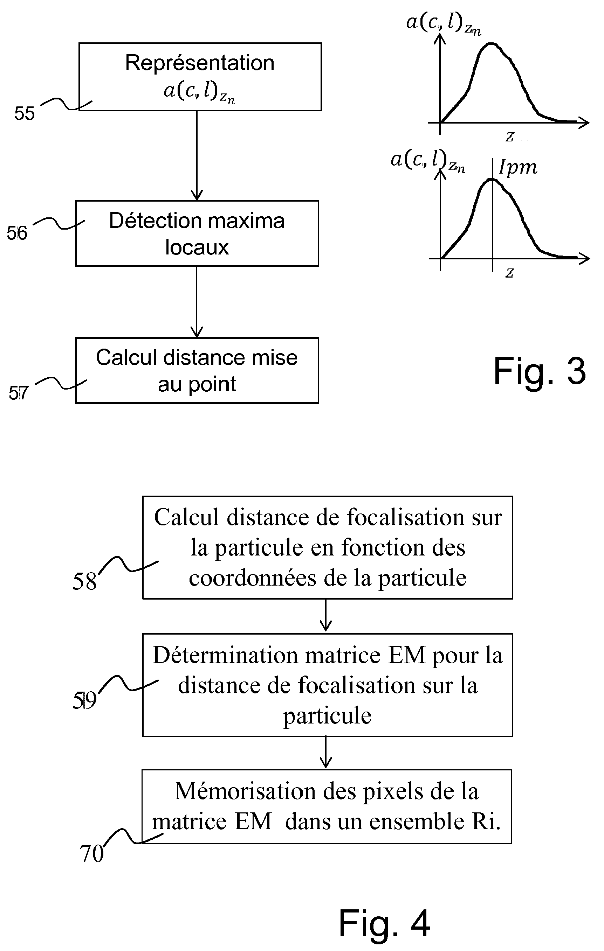

La figure 3 illustre un exemple de détermination de la distance de focalisation moyenne *zfmoy* en représentant, pour chaque coordonnée *(c, l),* la variation de $a(c, l)_{zn}$ en fonction de la distance *z* dans la pile d'images *I1 - IN.* Lorsqu'une particule est située dans l'échantillon sur l'axe, parallèle à l'axe optique du système **23**, de coordonnée (c,l) dans le plan focal, on observe une variation de $a(c, l)_{zn}$. Par exemple, la représentation, en **55**, des variations $a(c, l)_{zn}$ est analogue à une fonction gaussienne en fonction de la distance z. Au contraire, lorsqu'aucune particule n'est présente sur cet axe, signifiant que seul le milieu de l'échantillon est présent, $a(c, l)_{zn}$ ne varie pas ou très peu. Il est ainsi possible de détecter, en **56,** un optimum *Ipm* pour chaque coordonnée (c, 1) représentant la distance *z* de mise au point particulière pour cette coordonnée. La distance de focalisation moyenne *zfmoy* peut donc être recherchée par l'unité **20** en calculant, en **57,** la moyenne des distances *z* obtenues en détectant l'optimum *Ipm* de chaque coordonnée (c,l). *Ifmoy* est donc la matrice de la série de matrices *I1 - IN* la plus proche de la distance z calculée. En variante, l'unité **20** sélectionne les *P* coordonnées, par exemple les 10000 coordonnées, présentant les plus grandes variations de leurs valeurs $a(c, l)_{zn}$ en fonction de *z* (e.g. présentant les plus grands écarts entre la valeur maximale et la valeur minimale), puis calcule la distance *zfmoy* sur ces *P* coordonnées, ce qui augmente la précision sur cette distance. En variante, les valeurs $a(c, l)_{zn}$ de ces *P* coordonnées sont moyennées et l'optimum de la courbe moyenne en fonction de *z* est calculé, la distance z de l'optimum étant la distance *zfmoy*

Bien que la distance de focalisation moyenne *zfmoy* illustre une mise au point générale de l'image, la mise au point de chaque particule **11a-11f** n'est pas optimale, notamment en raison des variations de profondeur des particules **11a-11f** entre elles. Pour améliorer la mise au point d'une particule **11a-11f** particulière, l'invention propose de déterminer une distance de focalisation optimale pour chaque particule et de déterminer une matrice focalisée spécifique à la particule. A cet effet, l'unité de traitement **20** comporte un module **53** d'identification des particules et un module **54** de détermination d'une distance de focalisation optimale pour chaque particule identifiée et de détermination des pixels de la particule pour cette distance.

Le module **53** d'identification des particules dans la matrice *Ifmoy* peut prendre plusieurs formes de segmentation d'image de l'état de la technique, tel qu'un balayage de cette matrice jusqu'à détecter les contours d'un élément fini. En variante, l'unité **20** applique un seuillage préalable à la matrice *Ifmoy,* la valeur seuil étant par exemple égale à $Moy(Ifmoy) + p \times E(Ifmoy)$, où *Moy(Ifmoy)* est la moyenne des pixels de la matrice *Ifmoy, E(Ifmoy)* est leur écart-type, *et p* un entier supérieur à 1, par exemple égal à 6 Les valeurs supérieures à ce seuil sont alors déterminées comme appartenant à des particules, et une segmentation d'image sur la matrice seuillée est mise en oeuvre. A l'issue de l'identification, il est ainsi obtenu *I* ensembles de coordonnées de pixel (c,l), notés *Part_1,..., Part_i,... Part_I.* Chaque ensemble, mémorisé dans l'unité **20,** répertorie les coordonnées des pixels de l'image *Ifmoy* appartenant à une même particule.

Le procédé détermine ensuite, en **54,** pour chaque ensemble de coordonnées *Part_i,* quelle distance *z* offre la meilleure focalisation pour la particule correspondante, puis détermine quelle matrice de la série *I1 — IN* correspond à cette distance (ou calcule un nouvelle image pour cette distance), et finalement mémorise dans un ensemble **Ri** les pixels de la matrice sélectionnée qui correspondent aux coordonnées listés dans l'ensemble *Part_i.*

La figure 4 illustre un exemple de détermination d'un ensemble **R**i. Cette détermination débute, en **58,** par le calcul de la distance de focalisation optimale *zfopt_i,* par exemple de manière analogue au calcul de la distance de focalisation moyenne. Par exemple, pour chaque coordonnée (c,l) de l'ensemble *Part_i,* la distance correspondant à l'optimum de $a(c, l)_{zn}$ est calculée, puis la distance de focalisation optimale *zfopt_i* est choisie égale à la moyenne des distances **z** calculées. En variante, les valeurs $a(c, l)_{zn}$ de l'ensemble **Part_i** sont moyennées, et l'optimum de la courbe des valeurs moyennées en fonction de **z** est calculé, la distance **z** de l'optimum étant la distance **zfopt_i.** Ensuite, en **59,** la matrice de la série **I1 — IN** correspondant à la distance **zfopt_i,** notée **I_i,** est sélectionnée. En **70,** les pixels de la matrice sélectionnée de coordonnées listées dans l'ensemble **Part_i** sont sélectionnés et mémorisés dans un ensemble **Ri.**

En outre, les modes de réalisation des figures 3 et 4 sont facilement combinables car ils utilisent les mêmes fonctions gaussiennes. Les traitements peuvent donc être effectués en parallèle de sorte à limiter le temps de calcul. Le temps de calcul peut également être réduit en sous-échantillonnant (e.g. : en **z** à une période de $0,1\,\mu m$) les matrices de la pile de matrice **I1 — IN** permettant de sélectionner l'ensemble **Ri.**

Pour chaque particule identifiée, une image **Ifp_i** est préférentiellement affichée sur un écran **22.** Cette image est par exemple une fenêtre rectangulaire de la matrice **I_i** comprenant les pixels **Ri.** Comme illustré aux figures, une bactérie en bâtonnet a effectivement la forme d'un bâtonnet dans la matrice focalisée **I_i.** Un praticien peut ainsi observer la forme de la particule **11a-11f** très rapidement sans effectuer de manipulation sur le système optique **23.** Pour améliorer l'analyse de la particule **11a-11f,** celle-ci peut être représentée au cours du temps par le biais de plusieurs acquisitions successives.

Pour ce faire, lorsque l'ensemble **Ri** est obtenu, il est stocké dans une mémoire **21** reliée à l'unité de traitement **20.** Après une durée prédéfinie, un nouvel ensemble **Ri** est recherché par le biais d'une nouvelle acquisition d'image **Ih,** le pas de temps étant inférieur ou égal à 5 minutes dans le cadre de bactéries, et de préférence inférieure ou égale à 1 minute. Les inventeurs ont noté au travers de leur invention que cette période permet de suivre les modifications phénotypiques ou morphologiques de tout type de bactérie. En effet, on observe, par la simple inspection visuelle d'une série temporelle d'images **Ifp_i** d'une bactérie, que certaines bactéries changent d'une minute à l'autre.

Le nouvel ensemble **Ri** doit correspondre à la même particule **11a-11f** que le premier ensemble **Ri.** Pour ce faire, le module **53** de détermination des ensembles **Part_1 - Part_N** est réalisé en fonction de la position et/ou de la forme d'une particule **11a-11f** sélectionnée sur une matrice **Ifmoy** précédente. Les ensembles **Ri** focalisées **Ifp_i** sont tous stockés dans la mémoire **21** et l'écran **22** permet au praticien de lancer un flux d'images focalisées **Ifp_i** sur une particule **11a-11f** de sorte à analyser son comportement.

Le dispositif peut également réaliser l'analyse de plusieurs échantillons **12** simultanément, par exemple pour effectuer un antibiogramme d'une bactérie. Pour ce faire, l'unité de traitement **20** peut être reliée à plusieurs capteurs d'images **16** associés à plusieurs échantillons **12.** L'unité de traitement **20** effectue alors le traitement de plusieurs images **Ih** issues de plusieurs capteurs d'images **16** et l'écran **22** illustre les images focalisées **Ifp.**

Dans le mode de réalisation venant d'être décrit, la distance focale optimale $zfopt\_i$ correspond à une focalisation moyenne sur la particule, menant usuellement à une mise au point sur un plan médian de la particule. Ceci permet notamment de comparer les ensembles **Ri** entre plusieurs instants d'acquisition. En variante, une focalisation est recherchée pour chaque pixel (e.g. calcul de la distance correspondant à l'optimal de $a(c, l)_{zn}$ en fonction de **z,** recherche de la matrice électromagnétique à cette distance, mémorisation dans l'ensemble **Ri** du pixel de cette matrice de coordonnée (c,l)), ce qui permet de construire une visualisation 3D de la particule. Par contre, la comparaison entre deux reconstructions successives de la particule est plus difficile.

L'acquisition selon l'invention étant réalisée sans focus, on peut acquérir à une fréquence très élevée, et si besoin faire le traitement après avoir acquis.

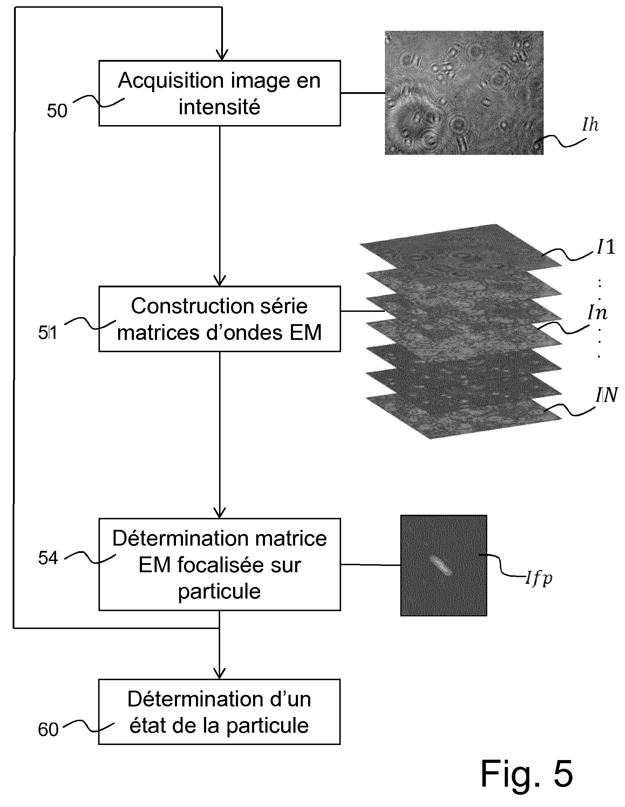

La figure 5 illustre un second mode de réalisation de l'invention consistant à détecter la division d'un microorganisme en fonction du temps. Dans ce mode de réalisation, l'unité de traitement **20** comporte un module de détermination **60** d'un état de la particule **11a-11f** en fonction de l'évolution temporelle de l'ensemble de pixels **Ri** associé au microorganisme, e.g. l'état phénotypique ou morphologique d'une bactérie.

Dans le mode de réalisation de la figure 5, la détermination **54** de l'ensemble de pixels **Ri** peut être effectuée par une simple recherche de focalisation numérique à partir de la pile d'images **I1 - IN** contrairement au mode de réalisation de la figure 2 dans lequel une double recherche de focalisation est effectuée. Les pixels de l'ensemble **Ri** sont alors ceux de la matrice **Ifmoy.** En variante, la détermination **54** de l'ensemble de pixels **Ri** peut être réalisée avec un processus analogue à celui de la figure 2.

Le module de détermination **60** d'un état de la particule **11a-11f** peut mettre en oeuvre des indicateurs et des traitements différents. Par exemple, l'état de la particule **11a-11f** peut être analysé selon l'état de son cycle de division, ses caractéristiques morphologiques, ses caractéristiques physiologiques ou une combinaison de plusieurs indicateurs.

De préférence, les indicateurs sont définis en fonction de la nature de la particule **11a-11f** dont l'état est recherché.

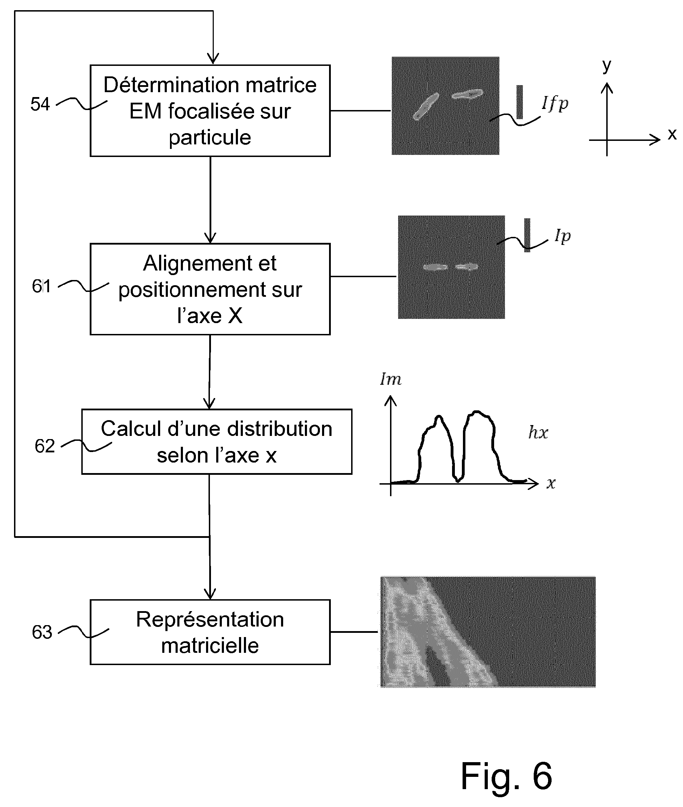

Pour une particule **11a-11f** analogue à une bactérie en présence d'un antibiotique, le mode de réalisation de la figure 6 est particulièrement efficace. Dans ce mode de réalisation, des images d'intensité **Ih** sont acquises périodiquement, avantageusement toute les minutes pour les raisons décrites plus haut, et pour chaque image **Ih** acquise, l'ensemble **Ri** associé à la bactérie est déterminé

Une rotation **61** est appliquée à l'ensemble **Ri** de sorte à aligner et positionner la particule **11a-11f** sur un axe prédéfini constant dans le temps, par exemple en alignant à gauche la particule sur l'axe *x* des abscisses (i.e l'axe correspondant aux lignes des images acquises). L'ensemble *Ri* aligné est ensuite projeté sur l'axe d'alignement, par exemple en réalisant la moyenne des pixels sur chaque ordonnée *y* (i.e. l'axe des colonnes des images acquises). La distribution *Ip* ainsi obtenue est représentée 62 sous la forme d'une distribution de la somme *Im* par colonne en fonction de la position de l'ordonné *x* des pixels, et est par exemple mémorisée dans l'unité **20** sous la forme d'une vecteur ligne.

Les distributions sont ensuite représentées au cours du temps en concaténant dans une matrice les distributions *Ip*. En notant *Ip_q* la distribution obtenue à partir de la qième image *Ih* acquise, et *Q* le dernier instant d'acquisition, la matrice, notée *Dist_Q,* s'écrit ainsi sous la forme :

$$Dist\_Q = \begin{pmatrix} Ip\_1 \\ \vdots \\ Ip\_q \\ \vdots \\ Ip\_Q \end{pmatrix}$$

La représentation matricielle ainsi obtenue est particulièrement efficace pour déterminer l'état de la particule **11a-11f**. Par exemple, cette représentation matricielle permet d'observer l'allongement d'une particule ou la présence d'au moins une division cellulaire.

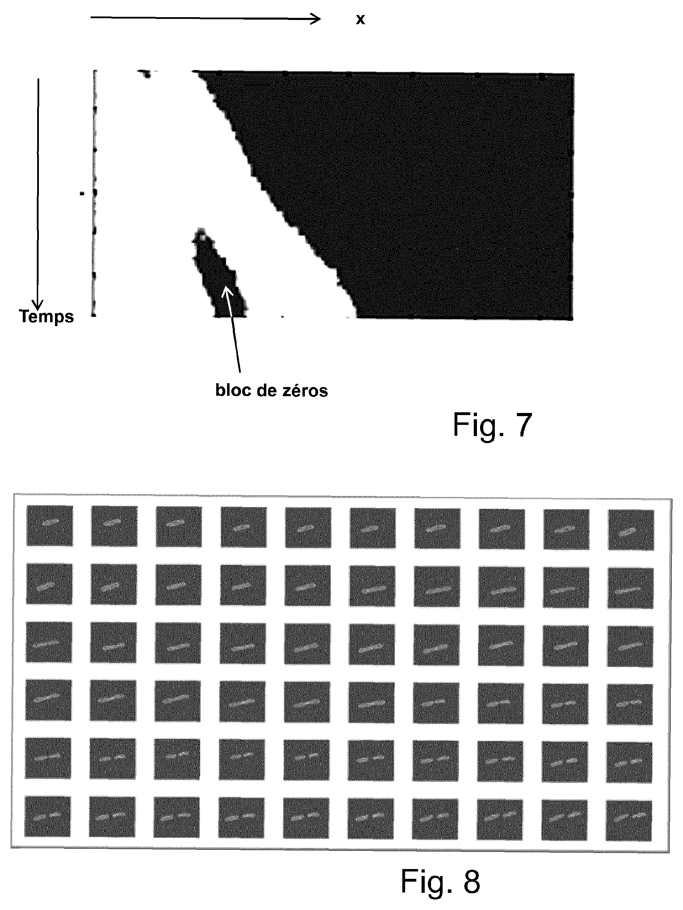

Un simple seuillage sur la forme de la représentation matricielle permet alors de définir si un antibiotique a agi efficacement ou non sur une bactérie. Notamment, comme illustré à la figure 7, on voit apparaitre dans la matrice seuillée, composée de zéro et de un, un bloc de zéros encadré par deux blocs de 1, l'apparition du bloc de zéros marquant l'instant de début de division, et s'accroitre. L'identification par l'unité de traitement **20** de l'apparition d'un tel bloc permet ainsi d'identifier d'une manière simple la division de la bactérie. En variante, d'autres analyses peuvent être effectuées en modélisant la vitesse de déplacement d'une particule **11a-11f** entre les images holographique *Ih* captée par le capteur d'image **16** ou en analysant les caractéristiques structurelles d'une particule **11a-11f** par correspondance avec des particules de forme connue.

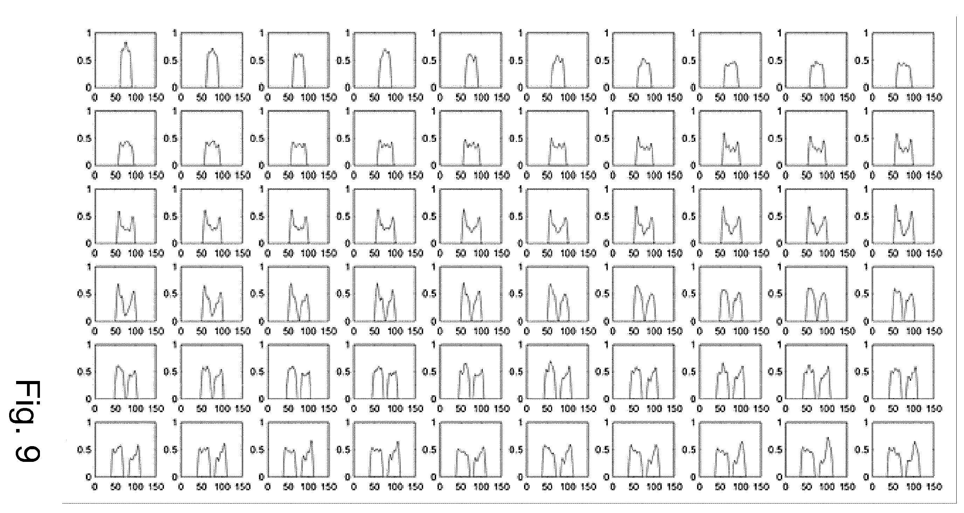

L'invention permet ainsi d'obtenir une image précise d'une particule **11a-11f** présente dans un échantillon **12**. Typiquement, l'invention a permis d'obtenir une imagette représentant une bactérie avec un nombre de pixels compris entre 100 et 400 pixels. Il s'ensuit qu'il est possible visuellement ou numériquement de visualiser, et ce sans marquage (e.g. fluorescent), le contenu d'un microorganisme. Notamment, on peut observer la répartition du matériel intracellulaire dans une bactérie. Sans être lié à la théorie, les inventeurs ont pu observer l'apparition de deux pôles dans une bactérie avant sa division, ce qui pourrait correspondre à la méiose d'une bactérie, comme illustré sur la figure 8 qui représente en fonction du temps l'évolution d'une bactérie. L'unité de traitement **20** met ainsi en oeuvre au cours du temps un traitement de l'ensemble *Ri* d'une bactérie, ou de la distribution *Ip_q,* afin de déterminer l'apparition de deux zones de valeur analogue. Une division de la bactérie est ainsi prédite bien avant que cette division n'ait lieu. La figure 9 illustre les distributions *Ip_q* de la bactérie illustrée à la figure 8. Comme on peut le constater à cette figure, lorsque la bactérie se prépare à la division, il apparait deux pôles dans la distribution, bien avant la division elle-même qui se traduit par deux portions distinctes de la distribution. L'unité de traitement **20** est par exemple configurée pour détecter l'apparition de deux pôles dans la distribution et prédire en fonction de cette détection la division de la bactérie, et par conséquent sa susceptibilité à l'antibiotique présent dans l'échantillon.

En outre, l'invention permet également d'obtenir l'évolution de la particule **11a-11f** au cours du temps afin de visualiser son comportement, par exemple sa vitesse de déplacement ou son processus de division cellulaire.

Notamment grâce à la résolution obtenue par l'invention (plusieurs centaines de pixels par bactérie pour système conçu par les inventeurs), il est possible d'observer l'influence d'un antibiotique sur la morphologie et le contenu intracellulaire des bactéries. En outre, la cadence élevée d'acquisition de l'invention permet traquer de manière très fine temporellement les variations de la bactérie (e.g. croissance, division, réaction à un milieu de culture, à un milieu avec antibiotique, etc.)

Il a été décrit un objectif dans l'air et à distance de l'échantillon. D'autres types d'agencement sont possibles, par exemple un objectif microscopique immergé dans une cuve de liquide recevant l'échantillon et/ou pouvant se déplacer par rapport à l'échantillon.

**Revendications**

1. Dispositif d'acquisition (10) d'une pluralité de particules (11a-11f) présentes dans un échantillon (12), ledit dispositif d'acquisition (10) comportant :

- une source lumineuse (15), spatialement cohérente ou pseudo-cohérente, orientée sur une première face (13) dudit échantillon (12) ;
- un système optique (23) ayant un axe optique et réalisant la conjugaison entre un plan de mise au point et un plan focal, orienté sur une seconde face (14) dudit échantillon (12) opposé à ladite première face (13), et placé par rapport à l'échantillon de sorte que les particules ne sont pas dans le plan de mis au point ;
- un capteur d'image (16), placé dans le plan focal du système optique et configuré pour acquérir une image en intensité (Ih) formée par l'interférence entre ladite source lumineuse (15) et ledit échantillon (12) ; et
- une unité de traitement informatique (20) comportant :

  o un module (51) de construction numérique d'une série de matrices électromagnétiques (I1 - IN) modélisant, à partir de l'image acquise (Ih), l'onde électromagnétique dans des plans parallèles au plan de mise au point et compris dans l'échantillon pour une pluralité d'écarts par rapport audit plan,
  o un module (52) de détermination d'une première matrice électromagnétique (Ifmoy) à une distance de mise au point moyenne sur les particules (11a-11f) à partir de la série de matrices électromagnétiques (I1 - IN) ;
  o un module (53) d'identification d'au moins une des particules (11a-11f) dans la première matrice électromagnétique (Ifmoy) et de mémorisation des coordonnées de ladite particule ; et
  o un module (54) de détermination d'une seconde matrice électromagnétique (Ifp) à une distance de mise au point sur une particule identifiée (11a-11f) à partir des composantes de la série de matrices électromagnétique (I1 - IN) ayant les coordonnées mémorisées.

**2.** Dispositif d'acquisition selon la revendication 1, *dans lequel* ledit capteur d'image (16) est configuré pour acquérir un flux d'images (Ih), et dans lequel l'unité de traitement est configurée pour suivre une particule dans le flux de premières matrices électromagnétiques *(Ifmoy).*

**3.** Dispositif d'acquisition selon la revendication 2, *dans lequel* les particules sont des microorganismes, et dans lequel la durée entre deux images acquises *(Ih)* issues dudit capteur d'image (16) est inférieure ou égale à 1 minute.

**4.** Dispositif d'acquisition selon l'une des revendications 1 à 3, *dans lequel* ladite série de matrices électromagnétiques *(I1 - IN)* est obtenue par un modèle de propagation numérique d'une lumière à travers ledit échantillon (12), les matrices électromagnétiques *(I1 - IN)* variant en modulant une distance (z) d'un axe optique dudit modèle de propagation.

**5.** Dispositif d'acquisition selon la revendication 4, *dans lequel* l'unité de traitement comprend un module de transformation des matrices électromagnétiques issues du modèle de propagation par une application surjective de l'espace complexe dans l'espace réel.

**6.** Dispositif d'acquisition selon la revendication 4 ou 5, *dans lequel* la première matrice électromagnétique (Ifmoy) est obtenue en représentant, à chaque coordonnée, les composantes à ladite coordonnée des matrices électromagnétiques *(I1 - IN)* en fonction de ladite distance (z) dudit axe optique, et en recherchant une moyenne des maxima de toutes les représentations.

**7.** Dispositif d'acquisition selon la revendication 6, *dans lequel* la seconde image électromagnétique est obtenue en représentant, à chaque coordonnée de la particule identifiée, les composantes à ladite coordonnée des matrices électromagnétiques *(I1 - IN)* en fonction de ladite distance (z) dudit axe optique, et en recherchant une moyenne des maxima de toutes les représentations.

**8.** Dispositif d'acquisition selon l'une des revendications précédentes, *dans lequel* les matrices de ladite série de matrices électromagnétiques *(I1 - IN)* utilisées pour déterminer ladite première matrices électromagnétiques (Ifmoy) et/ou pour déterminer ladite seconde matrice électromagnétique sont sous-échantillonnées selon ladite distance.

**9.** Dispositif d'acquisition selon l'une des revendications précédentes, *dans lequel* ledit dispositif comporte plusieurs unités d'acquisition, chaque unité d'acquisition comportant un capteur d'image et des moyens de focalisation spécifiques, ledit dispositif étant configuré pour représenter une image d'une particule de chaque échantillon.

**10.** Procédé d'analyse d'au moins une particule (11a-11f) présente dans un échantillon (12), ledit procédé d'analyse comportant les étapes suivantes :

- émission d'une source lumineuse (15), spatialement cohérente ou pseudo-cohérente, orientée sur une première face (13) dudit échantillon (12) ;
- acquisition (50) à l'aide d'un capteur d'image d'une image en intensité *(Ih),* ledit capteur étant placé dans le plan focal d'un système optique (23) ayant un axe optique et réalisant la conjugaison entre un plan de mise au point et le plan focal, orienté sur une seconde face (14) dudit échantillon (12) opposé à ladite première face (13), et placé par rapport à l'échantillon de sorte que la particule n'est pas dans le plan de mis au point, l'image (Ih) étant formée par interférence entre ladite source lumineuse (15) et ledit échantillon (12) ;
- construction numérique d'une série de matrices électromagnétiques (*I*1 - *I*N) modélisant, à partir de l'image acquise *(Ih),* l'onde électromagnétique dans des plans parallèles au plan de mise au point et compris dans l'échantillon pour une pluralité d'écarts par rapport audit plan ;
- obtention (54) d'une matrice électromagnétique (*Ifp*) à une distance de mise au point sur la particule (11a-11f) à partir de la série de matrices électromagnétique (*I*1 - *I*N) ; et
- détermination d'un état (60) de ladite particule (11a-11f) en fonction de la matrice électromagnétique (*Ifp*) à la distance de mise au point sur la particule (11a-11f).

**11.** Procédé d'analyse selon la revendication 10, *dans lequel* l'étape d'obtention la matrice électromagnétique (*Ifp*) à la distance de mise au point sur la particule (11a-11f) comporte les étapes suivantes :

- détermination (52) d'une première matrice électromagnétique (*Ifmoy*) à une distance de mise au point moyenne sur les particules (11a-11f) à partir de la série de matrices électromagnétiques (*I*1 - *I*N) ;
- l'identification (53) de la particule (11a-11f) dans la première matrice électromagnétique (*Ifmoy*) et la mémorisation des coordonnées de ladite particule ; et
- la détermination (54) de la matrice électromagnétique (*Ifp*) à la distance de mise au point sur la particule à partir des composantes de la série de matrices électromagnétique (*I*1 - IN) ayant les coordonnées mémorisées.

**12.** Procédé d'analyse selon la revendication 10 ou 11, *dans lequel* ladite étape de détermination d'un état de ladite particule (11a-11f) comporte les étapes suivantes :

- rotation (61) de la matrice électromagnétique (*Ifp*) à la distance de mise au point sur la particule de manière à aligner la particule sur un axe prédéfini ; et
- calcul (62) d'une distribution de la matrice alignée selon ledit axe prédéfini.

**13.** Procédé d'analyse selon l'une des revendications 10 à 12, *dans lequel* les étapes d'acquisition d'une image *(Ih)* jusqu'à l'étape d'obtention de la matrice électromagnétique (*Ifp*) à la distance de mise au point sur la particule sont réalisées au cours du temps pour plusieurs images, l'étape de détermination d'un état de ladite particule (11a-11f) étant réalisée en fonction d'une évolution temporelle de ladite particule (11a-11f).

**14.** Procédé d'analyse selon la revendication 13, *dans lequel* ladite étape de détermination d'un état de ladite particule (11a-11f) comporte les étapes suivantes :

- rotation (61), pour chaque image acquise, de la matrice électromagnétique (*Ifp*) à la distance de mise au point sur la particule de manière à aligner la particule sur un axe prédéfini ;
- calcul (62), pour chaque image acquise, d'une distribution de la matrice alignée selon ledit axe prédéfini ; et
- représentation (63) matricielle des distributions associées à chaque image en fonction du temps.

**15.** Procédé d'analyse selon l'une des revendications 10 à 14, *dans lequel* ladite étape de détermination (60) d'un état de ladite particule (11a-11f) est réalisée en fonction d'un état de division de ladite particule (11a-11f).

**16.** Procédé d'analyse selon l'une des revendications 10 à 15, *dans lequel* ladite étape de détermination (60) d'un état de ladite particule (11a-11f) est réalisée en fonction d'une caractéristique morphologique de ladite particule (11a-11f), par exemple la longueur.

**17.** Procédé d'analyse selon l'une des revendications 10 à 16, *dans lequel* ladite étape de détermination (60) d'un état de ladite particule (11a-11f) est réalisée en fonction d'une caractéristique physiologique de ladite particule (11a-11f), par exemple le nombre de noyau.

**18.** Procédé d'analyse selon l'une des revendications 10 à 17, *dans lequel* ladite particule (11a-11f) correspond à une bactérie et ledit échantillon (12) intègre un antibiotique.

Fig. 1

Fig. 2

$a(c,l)_{z_n}$

$a(c,l)_{z_n}$ $Ipm$

| Représentation $a(c,l)_{z_n}$ |
55

| Détection maxima locaux |
56

| Calcul distance mise au point |
57

## Fig. 3

| Calcul distance de focalisation sur la particule en fonction des coordonnées de la particule |
58

| Détermination matrice EM pour la distance de focalisation sur la particule |
59

| Mémorisation des pixels de la matrice EM dans un ensemble Ri. |
70

## Fig. 4

Acquisition image en
intensité

50

*Ih*

Construction série
matrices d'ondes EM

51

*I*1

*In*

*IN*

Détermination matrice
EM focalisée sur
particule

54

*Ifp*

Détermination d'un
état de la particule

60

# Fig. 5

54 — Détermination matrice EM focalisée sur particule

$Ifp$

y

x

61 — Alignement et positionnement sur l'axe X

$Ip$

62 — Calcul d'une distribution selon l'axe x

$Im$

$hx$

$x$

63 — Représentation matricielle

# Fig. 6

bloc de zéros

Fig. 7

Fig. 8

Fig. 9

Europäisches
Patentamt

European
Patent Office

Office européen
des brevets

## RAPPORT DE RECHERCHE EUROPEENNE

Numéro de la demande

EP 16 30 5625

### DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | WO 2016/075279 A1 (COMMISSARIAT À L ÉNERGIE ATOMIQUE ET AUX ÉNERGIES ALTERNATIVES [FR]; B) 19 mai 2016 (2016-05-19) * figure 1 * ----- | 1-18 | INV. G01N21/45 G03H1/00 G03H1/08 |
| A | JACOB P FUGAL ET AL: "Practical methods for automated reconstruction and characterization of particles in digital in-line holograms; Practical methods for automated digital particle hologram analysis", MEASUREMENT SCIENCE AND TECHNOLOGY, IOP, BRISTOL, GB, vol. 20, no. 7, 1 juillet 2009 (2009-07-01), page 75501, XP020160483, ISSN: 0957-0233 * page 8, colonne 2 - page 12, colonne 1 * ----- | 1-18 | |

DOMAINES TECHNIQUES RECHERCHES (IPC)

G01N
G03H

Le présent rapport a été établi pour toutes les revendications

| Lieu de la recherche | Date d'achèvement de la recherche | Examinateur |
|---|---|---|
| La Haye | 1 décembre 2016 | Quertemont, Eric |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

 .......................................................................................................

& : membre de la même famille, document correspondant

EPO FORM 1503 03.82 (P04C02)

**ANNEXE AU RAPPORT DE RECHERCHE EUROPEENNE
RELATIF A LA DEMANDE DE BREVET EUROPEEN NO.**

EP 16 30 5625

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

01-12-2016

| Document brevet cité au rapport de recherche | Date de publication | Membre(s) de la famille de brevet(s) | Date de publication |
|---|---|---|---|
| WO 2016075279 A1 | 19-05-2016 | FR 3028616 A1<br>WO 2016075279 A1 | 20-05-2016<br>19-05-2016 |

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 2603601 A

**Littérature non-brevet citée dans la description**

- **MYUNG K.KIM.** Principles and techniques of digital holographie microscopy. *SPIE Reviews,* Janvier 2010, vol. 1 (1
- **N. WU et al.** Three-dimensional identification of microorganisms using a digital holographic microscope. *Computational and Mathematical Methods in Medicine,* 2013
- **AHMED EL MALLAHI.** Automated threedimensional detection and classification of living organisms using digital holography microscopy with partial spatial coherent source: application to monitoring of drinking water resources. *Applied Optics,* Janvier 2013, vol. 52 (1
- **SANG-HYUK LEE et al.** Holographic microscopy of holographically trapped three-dimensional structures. *Optics Express,* 19 Février 2017, vol. 15 (4), 1505-1512